# EUROPEAN PATENT SPECIFICATION

(11) **EP 3 578 967 B1**
(45) Date of publication and mention of the grant of the patent: **01.05.2024**
(21) Application number: 18748514.9
(22) Date of filing: 29.01.2018
(51) Int. Cl.: G01N 29/07, G01B 17/00, G01N 29/265, G01N 29/28, G01N 29/06, G01N 29/22

(54) **POSITION CONTROL DEVICE, POSITION CONTROL METHOD, AND ULTRASOUND IMAGING SYSTEM**
POSITIONSSTEUERUNGSVORRICHTUNG, POSITIONSSTEUERUNGSVERFAHREN UND ULTRASCHALLBILDGEBUNGSSYSTEM
DISPOSITIF DE CONTRÔLE DE POSITION, PROCÉDÉ DE CONTRÔLE DE POSITION, ET SYSTÈME IMAGEUR À ULTRASONS

(30) Priority: 31.01.2017 JP 2017015187
(43) Date of publication of application: 11.12.2019
(73) Proprietor: Hitachi Power Solutions Co., Ltd., Hitachi-shi, Ibaraki 317-0073 (JP)
(72) Inventor: MURAI, Masakatsu, Hitachi-shi Ibaraki 317-0073 (JP); YAMATOYA, Naofumi, Hitachi-shi Ibaraki 317-0073 (JP); OONUKI, Katsuhiko, Hitachi-shi Ibaraki 317-0073 (JP); KUROSAWA, Kazuyoshi, Hitachi-shi Ibaraki 317-0073 (JP); GOU, Toshimasa, Hitachi-shi Ibaraki 317-0073 (JP)
(74) Representative: Strehl Schübel-Hopf & Partner
(86) International application number: PCT/JP2018/002794
(87) International publication number: WO 2018/143134

(56) References cited:
- JP-A- H04 230 849
- JP-A- 2003 322 646
- JP-A- 2003 322 646
- JP-A- 2009 204 327
- US-A- 5 335 547
- US-A- 5 608 690
- US-A1- 2011 000 299

## Description

### TECHNICAL FIELD

The present invention relates to a position control device, a position control method, and an ultrasonic imaging system.

### BACKGROUND ART

It is known an ultrasonic imaging device (SAT: Scanning Acoustic Tomograph) that scans a surface of a sample by an ultrasonic probe unit and images the surface of the sample based on a displacement of a reflected wave. When observing the sample with a curved surface by using the SAT, it is necessary to adjust a position of the ultrasonic probe unit in order to keep a distance between the ultrasonic probe unit and the surface of the sample approximately constant (focal distance).

For example, Patent Document 1 discloses a workpiece suction and fixing device in which a flexible workpiece is suctioned by a plurality of suction cups each including a base part and a lip part, and the workpiece is stably suctioned and fixed in a state of being immersed in water in a water tank, so that a defect inspection accuracy is improved.

In addition, for example, Patent Document 2 discloses an ultrasonic imaging device in which a focal depth of an ultrasonic probe unit is set to be wide, and a depth map of an observation position of a sample is acquired by a pre-scan, then, the focal depth of the ultrasonic probe unit is set to be narrow, and the sample is observed at a high resolution by a main scan including the observation position.

### PRIOR ART LITERATURE

### Patent Literature

Patent Document 1: Japanese Patent Application Publication No. 2013-170902
Patent Document 2: Japanese Patent Application Publication No. 2016-121951

An ultrasonic flaw inspection apparatus measuring the distance to a sample surface as well as the deviation from an ideal scanning position and adjusting the position of an ultrasonic probe in view of the measured deviation is disclosed in US 2011/000299 A1 and in JP 2003 322646 A. A further ultrasonic flaw detection apparatus using three ultrasonic probes for measuring distance and deviations simultaneously along different scanning lines in disclosed in US 5 335 547 A.

### SUMMARY OF THE INVENTION

### Problems to be Solved

However, for the workpiece suction and fixing device described in Patent Document 1, it is difficult to suction and fix a workpiece that is easily broken. In addition, when the ultrasonic imaging device described in Patent Document 2 is used, although a sample having a deeply curved surface or a tilted defect structure can be observed at a high resolution, it is necessary to scan the entire sample twice (pre-scan and main scan) in order to adjust the ultrasonic probe unit to an appropriate position, and measurement time and working cost are increased.

In other words, when the ultrasonic imaging device is used to observe a sample with a curved surface at a high resolution, there is a problem that the measurement time and the working cost are increased.

The invention is made to solve the above-described problem, and an object of the invention is to provide a position control device by which the measurement time and the working cost are reduced.

### Solution to Problem

In order to solve the above problem, the present invention suggests the position control device and method defined in the independent claims. Further advantageous features are set out in the dependent claims.

### Advantageous Effects

According to the invention, it is possible to provide a position control device by which measurement time and working cost are reduced.

### BRIEF DESCRIPTION OF DRAWINGS

FIG. 1 is a diagram showing a configuration example of an ultrasonic imaging system according to an embodiment of the invention;
FIG. 2 is a view illustrating an example of a scanning method performed by an ultrasonic probe unit according to the embodiment of the invention;
FIGS. 3A and 3B are each a conceptual diagram in a case where a measurement for calculating a deviation is performed at each measurement point according to the embodiment of the invention;
FIGS. 4A and 4B are each a graph showing a relationship between time and a displacement of a reflected wave according to the embodiment of the invention;
FIG. 5 is a diagram showing an operation of a gate circuit according to the embodiment of the invention;
FIGS. 6A and 6B are each a conceptual diagram showing a scan performed by the ultrasonic probe unit according to the embodiment of the invention; and
FIG. 7 is a flowchart showing an example of a control method performed by a position control device according to the embodiment of the invention.

### DESCRIPTION OF EMBODIMENTS

### «Configuration of Ultrasonic Imaging System»

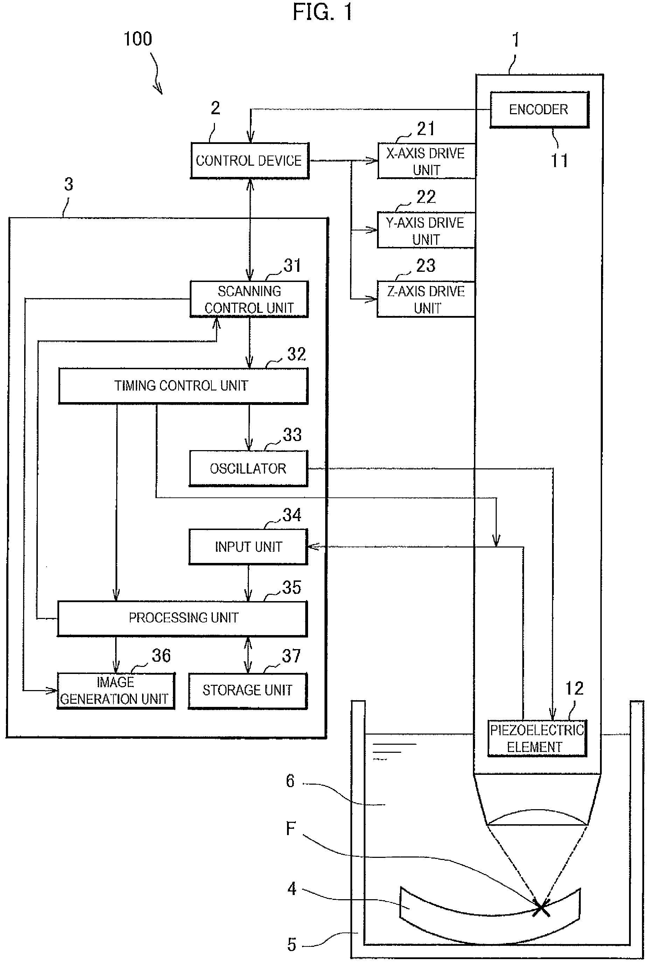

Hereinafter, an embodiment of the invention will be described with reference to the drawings. First, a configuration of an ultrasonic imaging system 100 according to the embodiment of the invention will be described with reference to FIG. 1.

As shown in FIG. 1, the ultrasonic imaging system 100 includes an ultrasonic probe unit 1, a control device 2, a position control device 3, an X-axis drive unit 21, a Y-axis drive unit 22, and a Z-axis drive unit 23.

In the ultrasonic imaging system 100, the ultrasonic probe unit 1 scans an entire surface of a sample 4, and the position control device 3 determines a position of the ultrasonic probe unit 1 such that a focal point F of the ultrasonic probe unit 1 matches the surface of the sample 4, and the control device 2 controls the position of the ultrasonic probe unit 1. The sample 4 is, for example, a disk-shaped silicon wafer with a curved surface. In the present embodiment, although a case is described as an example in which the disk-shaped silicon wafer with the curved surface is used as the sample 4, the sample to be observed using the ultrasonic imaging system 100 is not limited thereto.

The ultrasonic probe unit 1 includes an encoder 11 and a piezoelectric element 12. A lower part of the ultrasonic probe unit 1 is immersed in water 6 filled in a water tank 5.

The piezoelectric element 12 is provided to face the surface of the sample 4, and includes a piezoelectric film and electrodes formed on both surfaces of the piezoelectric film. When a voltage is applied between the two electrodes, the piezoelectric film vibrates, and an ultrasonic wave of a predetermined frequency is emitted from the piezoelectric element 12 to the surface of the sample 4. A reflected wave reflected by the surface of the sample 4 propagates to the piezoelectric element 12, and a voltage is generated between the two electrodes. The encoder 11 detects the position (position in ±X direction, position in ±Y direction, and position in ±Z direction) of the ultrasonic probe unit 1, and outputs a signal indicating the position of the ultrasonic probe unit 1 to the control device 2.

An output side of the control device 2 is connected to the X-axis drive unit 21, the Y-axis drive unit 22, and the Z-axis drive unit 23, and an input side of the control device 2 is connected to the position control device 3 and the encoder 11.

The control device 2 detects the position of the ultrasonic probe unit 1 (position in ±X direction, position in ±Y direction, and position in ±Z direction) based on the signal input from the encoder 11, and outputs a signal indicating the position of the ultrasonic probe unit 1 to the position control device 3.

The control device 2 controls the position of the ultrasonic probe unit 1 to the position determined by the position control device 3. That is, the control device 2 outputs, to the X-axis drive unit 21, a control signal for driving the ultrasonic probe unit 1 in the ±X direction based on a position signal input from the position control device 3. The control device 2 outputs, to the Y-axis drive unit 22, a control signal for driving the ultrasonic probe unit 1 in the ±Y direction based on the position signal input from the position control device 3. In addition, the control device 2 outputs, to the Z-axis drive unit 23, a control signal for driving the ultrasonic probe unit 1 in the ±Z direction based on the position signal input from the position control device 3.

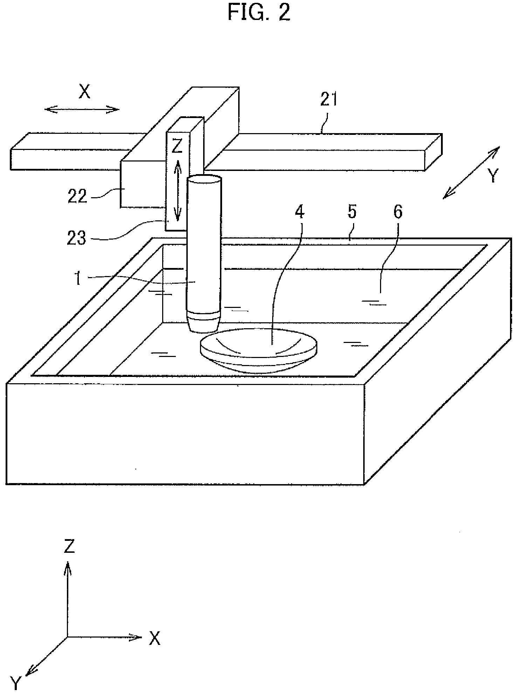

For example, as shown in FIG. 2, the ultrasonic probe unit 1 moves in the ±X direction by driving the X-axis drive unit 21 in a direction of an arrow, the ultrasonic probe unit 1 moves in the ±Y direction by driving the Y-axis drive unit 22 in a direction of an arrow, and the ultrasonic probe unit 1 moves in the ±Z direction by driving the Z-axis drive unit 23 in a direction of an arrow.

The position control device 3 includes a scanning control unit 31, a timing control unit 32, an oscillator 33, an input unit 34, a processing unit 35, an image generation unit 36, and a storage unit 37.

The scanning control unit 31 outputs, to the control device 2, the position signal for the control device 2 to control the position of the ultrasonic probe unit 1 in the ±X direction. The scanning control unit 31 outputs, to the control device 2, the position signal for the control device 2 to control the position of the ultrasonic probe unit 1 in the ±Y direction. In addition, the scanning control unit 31 outputs, to the control device 2, the position signal for the control device 2 to control the position of the ultrasonic probe unit 1 in the ±Z direction.

The scanning control unit 31 detects the position of the ultrasonic probe unit 1 (position in ±X direction, position in ±Y direction, and position in ±Z direction) based on the signal input from the control device 2, and outputs a signal indicating the position of the ultrasonic probe unit 1 to the timing control unit 32.

Based on the signal input from the scanning control unit 31, the timing control unit 32 generates a timing signal for controlling a timing at which the ultrasonic probe unit 1 transmits the ultrasonic wave to the surface of the sample 4, and outputs the timing signal to the oscillator 33. The timing control unit 32 generates a timing signal for controlling a timing at which the position control device 3 receives a reflection signal of the reflected wave reflected from the surface of the sample 4, and outputs the timing signal to the input unit 34. In addition, the timing control unit 32 generates a timing signal for controlling a timing at which the processing unit 35 performs gate processing on the reflection signal, and outputs the timing signal to the processing unit 35.

The oscillator 33 transmits an impulse signal to the ultrasonic probe unit 1 based on the timing signal input from the timing control unit 32. Accordingly, a voltage is applied between the two electrodes formed on the piezoelectric film, and the ultrasonic wave is emitted from the piezoelectric element 12 to the surface of the sample 4.

The input unit 34 includes an amplifier and an A/D converter, and receives the reflection signal of the reflected wave reflected from the surface of the sample 4 based on the timing signal input from the timing control unit 32, and outputs a reception signal to the processing unit 35. The amplifier amplifies the reflection signal and the A/D converter converts the reflection signal from an analog signal to a digital signal.

Based on the timing signal input from the timing control unit 32, the processing unit 35 performs gate processing on the reflection signal , detects a displacement of the reflected wave (for example, an amplitude information of the reflected wave, a time information of the reflected wave, and the like), and outputs the detection signal to the image generation unit 36. In addition, the processing unit 35 generates a tracking gate in order to cut out the displacement of the reflected wave in a predetermined period.

The processing unit 35 calculates a deviation (refer to description to be described later) when the ultrasonic probe unit 1 scans a predetermined line (for example, a third line) of the surface of the sample 4, and stores the calculated deviation in the storage unit 37. In addition, the processing unit 35 determines the position of the ultrasonic probe unit 1 in a direction (±Z direction) perpendicular to the surface of the sample 4 when the ultrasonic probe unit 1 is to scan a next line (for example, a fourth line) of the surface of the sample 4 such that the deviation is zero, and outputs the position signal to the scanning control unit 31. In the present specification, zero is not limited to being strictly zero, but substantially zero, and includes a range of measurement error from zero.

Detailed description of the deviation will be described later, and the deviation is a time difference between time (actual time) from transmitting the ultrasonic wave to the surface of the sample until the reflected wave is received and a reference time. In addition, the reference time is time from transmitting the ultrasonic wave to the surface of the sample until the reflected wave is received in a case where a distance between the ultrasonic probe unit 1 and the surface of the sample 4 is the same as a focal distance of the ultrasonic probe unit 1, and is time being a reference with respect to the actual time.

If the surface of the sample 4 is curved so as to approach the ultrasonic probe unit 1 (if the distance between the ultrasonic probe unit 1 and the surface of the sample 4 is shorter than the focal distance of the ultrasonic probe unit 1), the actual time is shorter, and the deviation is a positive value. In addition, if the surface of the sample 4 is curved so as to be away from the ultrasonic probe unit 1 (if the distance between the ultrasonic probe unit 1 and the surface of the sample 4 is longer than the focal distance of the ultrasonic probe unit 1), the actual time is longer, and the deviation is a negative value. That is, the distance between the ultrasonic probe unit 1 and the surface of the sample is dependent on the deviation (time difference).

Accordingly, the processing unit 35 determines the position of the ultrasonic probe unit 1 in the ±Z direction in accordance with a curvature of the surface of the sample 4, and thus high-resolution image can be generated in the ultrasonic imaging system 100.

In addition, a curvature of the surface of the sample 4 in a predetermined line and a curvature of the surface of the sample 4 in a next line are considered to be substantially equal. The processing unit 35 determines the position of the ultrasonic probe unit 1 when scanning a next line based on the deviation calculated for the predetermined line, which obtains substantially the same effect as determining the position of the ultrasonic probe unit 1 when scanning a next two line based on the deviation calculated for the next line. That is, substantially the same effect can be obtained as the processing unit 35 scanning a line in which the curvature of the surface of the sample is completely equal and determining the position of the ultrasonic probe unit. Accordingly, position correction of the ultrasonic probe unit 1 can be performed accurately in the ultrasonic imaging system 100.

In addition, the processing unit 35 sets measurement points at predetermined time intervals, and calculates a deviation for each measurement point.

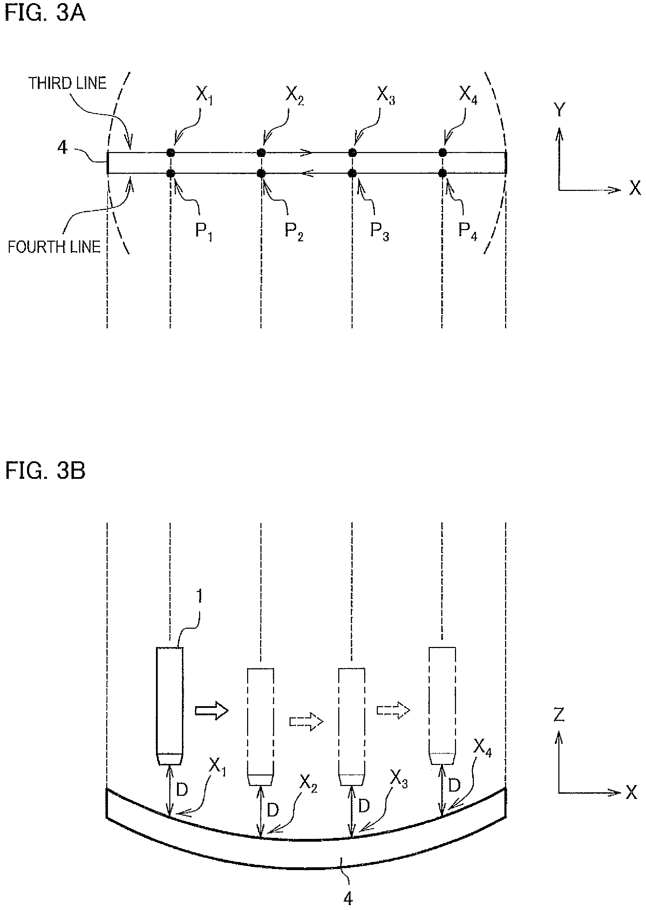

FIGS. 3A and 3B are each a conceptual diagram in a case where the ultrasonic probe unit 1 continuously travels in a +X direction and measurement for calculating a deviation is performed at each measurement point. FIG. 3A is a plan view showing a state in which the ultrasonic probe unit 1 scans the surface of the sample 4 in the +X direction when the measurement points set at predetermined time intervals are four points in the +X direction. FIG. 3B is a side view showing the state in which the ultrasonic probe unit 1 scans the surface of the sample 4 in the +X direction when the measurement points set at predetermined time intervals are four points in the +X direction.

X1 is a first measurement point of the predetermined line (for example, the third line). P1 is a point at which the position correction on the next line (for example, the fourth line) is performed based on the deviation calculated at the first measurement point X1, and is a point which is a first measurement point of the next line (for example, the fourth line) at the same time.

X2 is a second measurement point of the predetermined line (for example, the third line). P2 is a point at which the position correction on the next line (for example, the fourth line) is performed based on the deviation calculated at the second measurement point X2, and is a point which is a second measurement point of the next line (for example, the fourth line) at the same time.

X3 is a third measurement point of the predetermined line (for example, the third line). P3 is a point at which the position correction on the next line (for example, the fourth line) is performed based on the deviation calculated at the third measurement point X3, and is a point which is a third measurement point of the next line (for example, the fourth line) at the same time.

X4 is a fourth measurement point of the predetermined line (for example, the third line). P4 is a point at which the position correction on the next line (for example, the fourth line) is performed based on the deviation calculated at the fourth measurement point X4, and is a point which is a fourth measurement point of the next line (for example, the fourth line) at the same time.

For example, the processing unit 35 calculates a deviation at the first measurement point X1 on the predetermined line (for example, the third line) of the surface of the sample 4, and stores the deviation in the storage unit 37. Then, the processing unit 35 determines the position of the ultrasonic probe unit 1 in the ±Z direction at the point P1 of the next line (for example, the fourth line) based on the deviation calculated at the first measurement point X1.

Similarly, for example, the processing unit 35 calculates a deviation at the second measurement point X2 on the predetermined line (for example, the third line) of the surface of the sample 4, and stores the deviation in the storage unit 37. Then, the processing unit 35 determines the position of the ultrasonic probe unit 1 in the ±Z direction at the point P2 of the next line (for example, the fourth line) based on the deviation calculated at the second measurement point X2.

Similarly, for example, the processing unit 35 calculates a deviation at the third measurement point X3 on the predetermined line (for example, the third line) of the surface of the sample 4, and stores the deviation in the storage unit 37. Then, the processing unit 35 determines the position of the ultrasonic probe unit 1 in the ±Z direction at the point P3 of the next line (for example, the fourth line) based on the deviation calculated at the third measurement point X3.

Similarly, for example, the processing unit 35 calculates a deviation at the fourth measurement point X4 on the predetermined line (for example, the third line) of the surface of the sample 4, and stores the deviation in the storage unit 37. Then, the processing unit 35 determines the position of the ultrasonic probe unit 1 in the ±Z direction at the point P4 of the next line (for example, the fourth line) based on the deviation calculated at the fourth measurement point X4.

In other words, the processing unit 35 calculates a deviation for each measurement point of an (n-1)-th line (for example, the third line) set at predetermined time intervals. In addition, the processing unit 35 determines the position of the ultrasonic probe unit 1 in a direction (±Z direction) perpendicular to the surface of the sample for each measurement point of the n-th line (for example, the fourth line) such that the deviation calculated at a corresponding measurement point of the (n-1)-th line (for example, the third line) is zero.

A distance between the first measurement point X1 and the point P1 is extremely small. Therefore, for example, determining the position of the ultrasonic probe unit 1 in the ±Z direction at the point P1 on the fourth line based on the deviation calculated at the first measurement point X1 on the third line can be considered to be substantially the same as determining the position of the ultrasonic probe unit 1 in the ±Z direction at the point P1 on the fourth line based on a deviation calculated at the point P1 on the fourth line. Accordingly, when the ultrasonic probe unit 1 scans the fourth line, a distance D between the ultrasonic probe unit 1 and the surface of the sample 4 can be kept constant.

Similarly, a distance between the second measurement point X2 and the point P2 is extremely small. Therefore, for example, determining the position of the ultrasonic probe unit 1 in the ±Z direction at the point P2 on the fourth line based on the deviation calculated at the second measurement point X2 on the third line can be considered to be substantially the same as determining the position of the ultrasonic probe unit 1 in the ±Z direction at the point P2 on the fourth line based on a deviation calculated at the point P2 on the fourth line. Accordingly, when the ultrasonic probe unit 1 scans the fourth line, the distance D between the ultrasonic probe unit 1 and the surface of the sample 4 can be kept constant.

Similarly, a distance between the third measurement point X3 and the point P3 is extremely small. Therefore, for example, determining the position of the ultrasonic probe unit 1 in the ±Z direction at the point P3 on the fourth line based on the deviation calculated at the third measurement point X3 on the third line can be considered to be substantially the same as determining the position of the ultrasonic probe unit 1 in the ±Z direction at the point P3 on the fourth line based on a deviation calculated at the point P3 on the fourth line. Accordingly, when the ultrasonic probe unit 1 scans the fourth line, the distance D between the ultrasonic probe unit 1 and the surface of the sample 4 can be kept constant.

Similarly, a distance between the fourth measurement point X4 and the point P4 is extremely small. Therefore, for example, determining the position of the ultrasonic probe unit 1 in the ±Z direction at the point P4 on the fourth line based on the deviation calculated at the fourth measurement point X4 on the third line can be considered to be substantially the same as determining the position of the ultrasonic probe unit 1 in the ±Z direction at the point P4 on the fourth line based on a deviation calculated at the point P4 on the fourth line. Accordingly, when the ultrasonic probe unit 1 scans the fourth line, the distance D between the ultrasonic probe unit 1 and the surface of the sample 4 can be kept constant.

For the interval at which the measurement is performed for calculating a deviation when the ultrasonic probe unit 1 scans a predetermined line of the surface of the sample 4, it is possible to increase the interval at which the measurement is performed at a part where the surface of the sample 4 is less concave-convexed and the curvature is small, and to reduce the interval at which the measurement is performed at a part where the surface of the sample 4 is more concave-convexed and the curvature is large. In the part where the curvature is large, the measurement is performed intensively and the deviation is calculated, so that the position correction can be performed efficiently in the ultrasonic imaging system 100.

The image generation unit 36 generates an image based on a signal input from the processing unit 35. As described above, since the position of the ultrasonic probe unit 1 in the ±Z direction is adjusted to an appropriate position (a position where the focal point F of the ultrasonic probe unit 1 matches the surface of the sample 4), the image generation unit 36 can generate an image with a high image resolution.

The storage unit 37 stores the deviation calculated by the processing unit 35 when the ultrasonic probe unit 1 scans the predetermined line of the surface of the sample 4. In addition, the storage unit 37 stores the deviation used by the processing unit 35 when the ultrasonic probe unit 1 scans the next line of the surface of the sample 4. Therefore, the storage unit 37 only needs to have a memory capacity that stores deviations for at least two lines.

The storage unit 37 stores a predetermined reference time set in advance. In addition, the storage unit 37 stores a start point of the tracking gate, an end point of the tracking gate, a point where a voltage waveform has a peak, a point where a reference voltage waveform has a peak, and the like.

According to the position control device 3 according to the present embodiment, the storage unit stores the deviation when the ultrasonic probe unit scans a predetermined line of the surface of the sample, and the processing unit determines the position of the ultrasonic probe unit when the ultrasonic probe unit scans the next line of the surface of the sample such that the deviation is zero. Accordingly, the measurement time and the working cost can be reduced.

According to the ultrasonic imaging system 100 according to the present embodiment, the ultrasonic probe unit 1 can be adjusted to an appropriate position only by scanning the entire sample once. Accordingly, the measurement time and the working cost can be reduced. In addition, it is possible to improve the problem in the related art that it is necessary for the ultrasonic probe unit 1 to scan the entire sample twice in order to observe the sample at a high resolution.

### «Deviation Calculation»

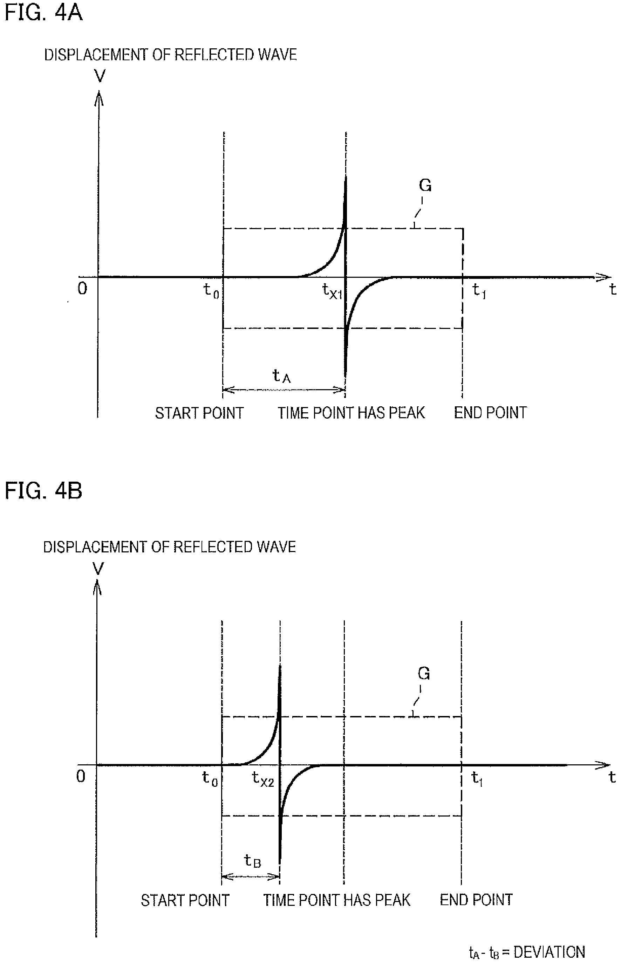

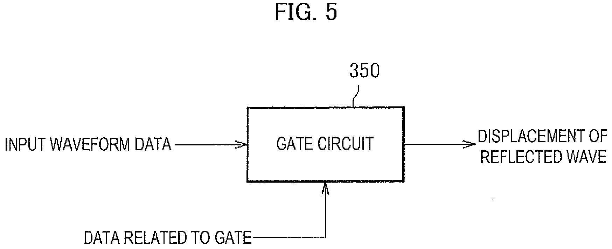

Next, the deviation described above will be described with reference to FIGS. 4A, 4B and FIG. 5. FIGS. 4A and 4B are each a graph showing a relationship between time and a displacement of the reflected wave. FIG. 4A shows the reference voltage waveform, and FIG. 4B shows an actually measured voltage waveform. A horizontal axis represents time [s], and a vertical axis represents voltage (displacement of reflected wave) [V].

The processing unit 35 generates a tracking gate G, plots the displacement of the reflected wave in the tracking gate G, compares the reference voltage waveform in the tracking gate G with the actually measured voltage waveform in the tracking gate G, and calculates the deviation.

As shown in FIG. 4A, a time point of a start point of the tracking gate G is set as t₀[s], a time point of an end point of the tracking gate G is set as t₁[s], a time point when the reference voltage waveform has a peak is set as tₓ₁[s], and time from the start point of the tracking gate G until the reference voltage waveform has a peak is set as t_{A}[s] (predetermined reference time).

As shown in FIG. 4B, the time point of the start point of the tracking gate G is set as t₀[s], the time point of the end point of the tracking gate G is set as t₁[s], a time point when the actually measured voltage waveform has a peak is set as tₓ₂[s], and time from the start point of the tracking gate G until the actually measured voltage waveform has a peak is set as t_{B}[s] (predetermined actual time).

The processing unit 35 calculates, a time difference (t_{A}-t_{B}[s]) between the time (t_{A}[s]) from the start point of the tracking gate until the reference voltage waveform has a peak, and the time (t_{B}[s]) from the start point of the tracking gate until the actually measured voltage waveform has a peak, as a deviation. That is, the processing unit 35 can generate a tracking gate, cut out the displacement of the reflected wave in a predetermined period, and calculate the deviation. The tracking gate G is arbitrarily set by the processing unit 35.

FIG. 5 is a diagram showing an operation of a gate circuit 350 provided in the processing unit 35.

As shown in FIG. 5, the gate circuit 350 receives waveform data in which the reflected wave is captured and data related to the gate to perform gate processing, and outputs the displacement of the reflected wave. The data related to the gate includes a start timing of the gate, a width of the gate, and a determination threshold value. The gate circuit 350 detects the displacement of the reflected wave exceeding the determination threshold value with respect to the reflected wave in the gate set in advance.

### «Scan of Ultrasonic Probe Unit»

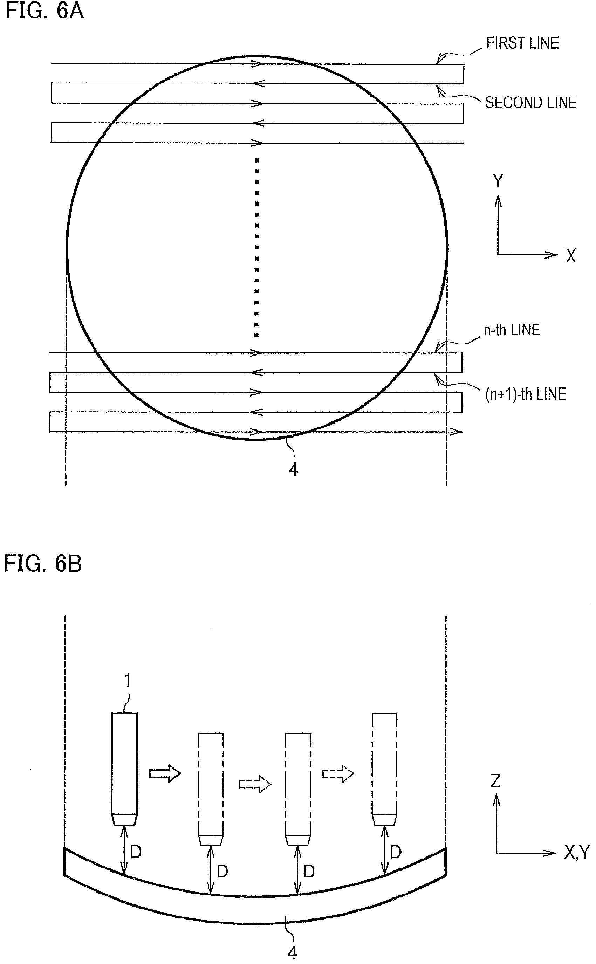

Next, the scan performed by the ultrasonic probe unit 1 will be described with reference to FIGS. 6A and 6B.

FIG. 6A is a plan view when the ultrasonic probe unit 1 scans the surface of the sample 4 in the ±X direction. FIG. 6B is a side view when the ultrasonic probe unit 1 scans the surface of the sample 4 in the ±X direction.

As shown in FIG. 6A, first, the ultrasonic probe unit 1 scans a first line of the surface of the sample in the +X direction. The processing unit 35 calculates deviations in the first line, and the storage unit 37 stores the deviations in the first line. When the ultrasonic probe unit 1 scans the first line of the surface of the sample, since the deviation for determining the position of the ultrasonic probe unit 1 in the ±Z direction has not yet been calculated, the ultrasonic probe unit 1 scans the first line of the surface of the sample without changing the position of the ultrasonic probe unit 1 in the ±Z direction.

Next, the ultrasonic probe unit 1 moves in a -Y direction by a predetermined pitch. The pitch is an extremely short distance (specifically, 0.01 mm or less). Therefore, a curvature of the surface of the sample in the first line and a curvature of the surface of the sample in a second line can be substantially equal.

Next, the ultrasonic probe unit 1 scans the second line of the surface of the sample in a -X direction. The processing unit 35 determines the position of the ultrasonic probe unit 1 in the ±Z direction when the ultrasonic probe unit 1 scans the second line of the surface of the sample in the -X direction based on the deviations in the first line stored in the storage unit 37. The scanning control unit 31 outputs a position signal for controlling the position of the ultrasonic probe unit 1 in the ±Z direction to the control device 2, and the control device 2 adjusts the position of the ultrasonic probe unit 1 in the ±Z direction to an appropriate position. The appropriate position is, as shown in FIG. 6B, a position where the distance D between the ultrasonic probe unit 1 and the surface of the sample 4 is constant.

In addition, the processing unit 35 calculates the deviations in the second line, and the storage unit 37 stores the deviations in the second line. At this time, the storage unit 37 stores the deviations in the first line and the deviations in the second line.

Next, the ultrasonic probe unit 1 moves in the -Y direction by a predetermined pitch. The pitch is an extremely short distance (specifically, 0.01 mm or less). Therefore, the curvature of the surface of the sample in the second line and a curvature of the surface of the sample in a third line can be substantially equal.

Next, the ultrasonic probe unit 1 scans the third line of the surface of the sample in the +X direction. The processing unit 35 determines the position of the ultrasonic probe unit 1 in the ±Z direction when the ultrasonic probe unit 1 scans the third line of the surface of the sample in the +X direction based on the deviations in the second line stored in the storage unit 37. The scanning control unit 31 outputs a position signal for controlling the position of the ultrasonic probe unit 1 in the ±Z direction to the control device 2, and the control device 2 adjusts the position of the ultrasonic probe unit 1 in the ±Z direction to an appropriate position. The appropriate position is, as shown in FIG. 6B, a position where the distance D between the ultrasonic probe unit 1 and the surface of the sample 4 is constant.

In addition, the processing unit 35 calculates the deviations in the third line, and the storage unit 37 stores the deviations in the third line. At this time, the storage unit 37 may store all of the deviations in the first line, the deviations in the second line, and the deviations in the third line, and may only store the deviations in the second line and the deviations in the third line. At least two lines (the deviations in the second line and the deviations in the third line) may be stored.

Next, the ultrasonic probe unit 1 moves in the -Y direction by a predetermined pitch. The pitch is an extremely short distance (specifically, 0.01 mm or less). Therefore, the curvature of the surface of the sample in the third line and a curvature of the surface of the sample in a fourth line can be substantially equal.

The ultrasonic probe unit 1 repeats the scan as described above, and scans all lines of the surface of the sample 4. Accordingly, an image of the surface of the sample 4 is generated.

According to the ultrasonic imaging system 100 according to the present embodiment, even for a sample with a curved surface, only by scanning the entire sample once by the ultrasonic probe unit, the distance D between the ultrasonic probe unit and the surface of the sample can be kept constant, and an image can be generated. Accordingly, it is possible to provide the ultrasonic imaging system 100 by which the measurement time and the working cost are reduced while maintaining the image resolution of the image compared to the case in which the entire sample is scanned twice by the ultrasonic probe unit.

In an ultrasonic imaging system in the related art, since the ultrasonic probe unit scans the entire sample twice, the number of mechanical processing is doubled as compared with the ultrasonic imaging system 100 according to the embodiment. Meanwhile, in the ultrasonic imaging system 100 according to the embodiment, since the processing unit determines the position of the ultrasonic probe unit while calculating the deviation, a processing amount of the processing unit is doubled as compared with the ultrasonic imaging system in the related art. However, the measurement time and the working cost in a case of performing the data processing twice are extremely small as compared with the measurement time and the work cost in a case of performing the scan processing twice. Therefore, as compared with the ultrasonic imaging system in the related art, the ultrasonic imaging system 100 according to the embodiment can reduce the measurement time and the working cost significantly.

### «Operation of Position Control Device»

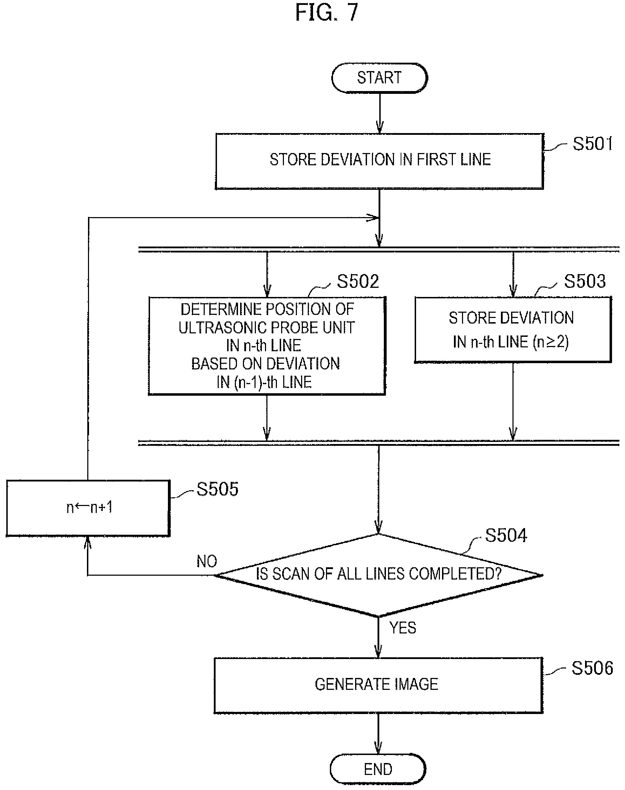

Next, the operation of the position control device 3 according to the embodiment of the invention will be described with reference to FIGS. 6A, 6B and FIG. 7.

FIG. 7 is a flowchart showing the operation of the position control device 3 according to the embodiment of the invention.

In step S501, the processing unit 35 calculates deviations when the ultrasonic probe unit 1 scans the first line of the surface of the sample 4, and the storage unit 37 stores the deviations.

In step S502, the processing unit 35 determines the position of the ultrasonic probe unit 1 when the ultrasonic probe unit 1 scans the second line (the n-th line) of the surface of the sample 4 based on the deviations in the first line ((n-1)-th line). N (n≥2) is a variable indicating a line number.

In step S503, the processing unit 35 calculates deviations when the ultrasonic probe unit 1 scans the second line (the n-th line) of the surface of the sample 4, and the storage unit 37 stores the deviations.

In step S504, the processing unit 35 determines whether the ultrasonic probe unit 1 has completed the scan of all lines of the surface of the sample 4. If it is determined that the ultrasonic probe unit 1 has not completed the scan of all lines of the surface of the sample 4 (step S504→No), the processing unit 35 performs processing of step S505. Meanwhile, if it is determined that the ultrasonic probe unit 1 has completed the scan of all lines of the surface of the sample 4 (step S504-->Yes), the processing unit 35 performs processing of step S506.

In step S505, the processing unit 35 increases the variable n (n≥2) indicating the line number by one. That is, n is increased from 2 to 3.

Again, in step S502, the processing unit 35 determines the position of the ultrasonic probe unit 1 when the ultrasonic probe unit 1 scans the third line (the n-th line) of the surface of the sample 4 based on the deviations in the second line ((n-1)-th line).

Again, in step S503, the processing unit 35 calculates deviations when the ultrasonic probe unit 1 scans the third line (the n-th line) of the surface of the sample 4, and the storage unit 37 stores the deviations.

Again, in step S504, the processing unit 35 determines whether the ultrasonic probe unit 1 has completed the scan of all lines of the surface of the sample 4. If it is determined that the ultrasonic probe unit 1 has not completed the scan of all lines of the surface of the sample 4 (step S504→No), the processing unit 35 performs processing of step S505. Meanwhile, if it is determined that the ultrasonic probe unit 1 has completed the scan of all lines of the surface of the sample 4 (step S504→Yes), the processing unit 35 performs processing of step S506.

Again, in step S505, the processing unit 35 increases the variable n (n≥2) indicating the line number by one. That is, n is increased from 3 to 4.

The processing from steps S502 to S505 is repeated as described above until the ultrasonic probe unit 1 completed the scan of all lines of the surface of the sample 4.

In step S506, the image generation unit 36 generates an image on the surface of the sample 4, and completes a series of processing.

According to the position control method described above, the measurement time and the working cost can be reduced.

### REFERENCE SIGN LIST

100: ultrasonic imaging system
1: ultrasonic probe unit
2: control device
3: position control device
35: processing unit
37: storage unit

## Claims

1. An ultrasonic imaging system, comprising:
an ultrasonic probe unit (1) that is configured to transmit an ultrasonic wave to a surface of a sample (4) and receive a reflected wave from the surface of the sample;
a position control device (3); and
a control device (2);
wherein the position control device, comprises:
a storage unit (37) configured to store a deviation between the time from transmitting an ultrasonic wave from the ultrasonic probe unit to the sample until a reflected wave is received and a reference time, the reference time being the time from transmitting the ultrasonic wave until the reflected wave is received when the distance between the ultrasonic probe unit and the surface of the sample is equal to the focal distance of the ultrasonic probe unit;
a processing unit (35) configured to determine a position of the ultrasonic probe unit in a direction perpendicular to the surface of the sample and to calculate a deviation between the time from transmitting an ultrasonic wave until a reflected wave is received and the reference time;
wherein the storage unit is configured to store the deviation in an (n-1)-th line as previously calculated by the processing unit when the ultrasonic probe unit scans the (n-1)-th line of a surface of a sample in a first surface direction of the sample before the ultrasonic probe unit moves by a predetermined pitch in a second surface direction perpendicular to the first surface direction in order to scan an n-th line of the surface of the sample in the surface direction opposite to the first surface direction; and
wherein the processing unit is configured to determine the position of the ultrasonic probe unit in the direction perpendicular to the surface of the sample when the ultrasonic probe unit scans the n-th line of the surface of the sample based on the deviation in the (n-1)-th line stored in the storage unit,
wherein the control device is configured to control the position of the ultrasonic probe unit in a direction perpendicular to the surface of the sample to the position determined by the position control device when the ultrasonic probe unit scans the n-th line of the surface of the sample; and
wherein the processing unit is configured to to calculate a deviation in the n-th line between the time from transmitting an ultrasonic wave until a reflected wave is received and the reference time when the ultrasonic probe unit scans the n-th line of the surface of the sample, and to store the calculated deviation in the storage unit.

2. The ultrasonic imaging system according to claim 1,
wherein the processing unit (35) is configured to calculate the deviation for each measurement point set at predetermined time intervals, and to make the storage unit (37) to store the deviation.

3. The ultrasonic imaging system according to claim 1 or 2,
wherein the processing unit (35) is configured to determine the position of the ultrasonic probe unit (1) in a direction perpendicular to the surface of the sample (4) when the ultrasonic probe unit scans the n-th line of the surface of the sample, such that the deviation that has been calculated for the (n-1)-th line and applies to the n-th line as well under the assumption of substantially equal surface curvatures in the (n-1)-th and n-th lines, is zero in the n-th line.

4. The ultrasonic imaging system according to any one of claims 1 to 3,
wherein the processing unit (35) is configured to:
generate a tracking gate, and
calculate the deviation by comparing time from a start point of the tracking gate until an actually measured voltage waveform and time from the start point of the tracking gate until a reference voltage waveform has a peak corresponding to said reference time.

5. A position control method, comprising:
a step of calculating and storing a deviation in an (n-1)-th line between time from transmitting an ultrasonic wave from an ultrasonic probe unit (1) to a sample (4) until a reflected wave is received and a reference time when the ultrasonic probe unit (1) scans the (n-1)-th line of a surface of the sample in a first surface direction of the sample, the reference time being the time from transmitting the ultrasonic wave until the reflected wave is received when the distance between the ultrasonic probe unit and the surface of the sample is equal to the focal distance of the ultrasonic probe unit;
a step (S505) of moving the ultrasonic probe unit by a predetermined pitch in a second surface direction perpendicular to the first surface direction in order to scan an n-th line of the surface of the sample in the surface direction opposite to the first surface direction;
a step (S502) of determining a position of the ultrasonic probe unit in a direction perpendicular to the surface of the sample when the ultrasonic probe unit scans the n-th line of the surface of the sample based on the stored deviation in the (n-1)-th line;
a step of scanning the n-th line of the surface of the sample by the ultrasonic probe unit while controlling the position of the ultrasonic probe unit to the determined position; and
returning (S505) to the above calculating and storing step in order to calculate the deviation in the n-th line between the time from transmitting an ultrasonic wave until a reflected wave is received and the reference time when the ultrasonic probe unit scans the n-th line of the surface of the sample, and to store the calculated deviation.

6. The position control method according to claim 5,
wherein in the step of determining a position of the ultrasonic probe unit (1) when the ultrasonic probe unit scans the n-th line of the surface of the sample (4), the position of the ultrasonic probe unit in a direction perpendicular to the surface of the sample (4) is determined such that the deviation that has been calculated for the (n-1)-th line and applies to the n-th line as well under the assumption of substantially equal surface curvatures in the (n-1)-th and n-th lines, is zero in the n-th line.

## Patentansprüche

1. Ultraschall-Bildgebungssystem, umfassend:
eine Ultraschallsondeneinheit (1), die dazu eingerichtet ist, eine Ultraschallwelle zu einer Oberfläche einer Probe (4) zu sende und eine von der Oberfläche der Probe reflektierte Welle zu empfangen;
eine Positionssteuervorrichtung (3); und
eine Steuervorrichtung (2);
wobei die Positionssteuervorrichtung umfasst:
eine Speichereinheit (37), die dazu eingerichtet ist, eine Abweichung zwischen der Zeit vom Senden einer Ultraschallwelle von der Ultraschallsondeneinheit zur Probe bis zum Empfang einer reflektierten Welle und einer Referenzzeit zu speichern, wobei die Referenzzeit die Zeit vom Senden der Ultraschallwelle bis zum Empfang der reflektierten Welle ist, wenn der Abstand zwischen der Ultraschallsondeneinheit und der Oberfläche der Probe gleich der Brennweite der Ultraschallsondeneinheit ist;
eine Verarbeitungseinheit (35), die dazu eingerichtet ist, eine Position der Ultraschallsondeneinheit in einer Richtung senkrecht zur Oberfläche der Probe zu bestimmen und eine Abweichung zwischen der Zeit vom Senden einer Ultraschallwelle bis zum Empfang einer reflektierten Welle und der Referenzzeit zu berechnen;
wobei die Speichereinheit dazu eingerichtet ist, die Abweichung in einer (n-1)-ten Linie, wie sie zuvor von der Verarbeitungseinheit berechnet wurde, zu speichern, wenn die Ultraschallsondeneinheit die (n-1)-te Linie einer Oberfläche einer Probe in einer ersten Oberflächenrichtung der Probe abtastet, bevor sich die Ultraschallsondeneinheit um einen vorbestimmten Intervallabstand in einer zweiten Oberflächenrichtung senkrecht zur ersten Oberflächenrichtung bewegt, um eine n-te Linie der Oberfläche der Probe in der Oberflächenrichtung entgegengesetzt der ersten Oberflächenrichtung abzutasten; und
wobei die Verarbeitungseinheit dazu eingerichtet ist, Position der Ultraschallsondeneinheit in der Richtung senkrecht zur Oberfläche der Probe, wenn die Ultraschallsondeneinheit die n-te Linie der Oberfläche der Probe abtastet, basierend auf der in der Speichereinheit gespeicherten Abweichung in der (n-1)-ten Linie zu bestimmen;
wobei die Steuervorrichtung dazu eingerichtet ist, die Position der Ultraschallsondeneinheit in einer Richtung senkrecht zur Oberfläche der Probe auf die von der Positionssteuervorrichtung bestimmte Position zu steuern, wenn die Ultraschallsondeneinheit die n-te Linie der Oberfläche der Probe abtastet; und
wobei die Verarbeitungseinheit dazu eingerichtet ist, eine Abweichung in der n-ten Linie zwischen der Zeit vom Senden einer Ultraschallwelle bis zum Empfang einer reflektierten Welle und der Referenzzeit zu berechnen, wenn die Ultraschallsondeneinheit die n-te Linie der Oberfläche der Probe abtastet, und die berechnete Abweichung in der Speichereinheit zu speichern.

2. Ultraschall-Bildgebungssystem nach Anspruch 1,
wobei die Verarbeitungseinheit (35) dazu eingerichtet ist, die Abweichung für jeden in vorgegebenen Zeitintervallen gesetzten Messpunkt berechnet und die Speichereinheit (37) die Abweichung speichern zu lassen.

3. Ultraschall-Bildgebungssystem nach Anspruch 1 oder 2,
wobei die Verarbeitungseinheit (35) dazu eingerichtet ist, die Position der Ultraschallsondeneinheit (1) in einer Richtung senkrecht zur Oberfläche der Probe (4) zu bestimmen, wenn die Ultraschallsondeneinheit die n-te Linie der Oberfläche der Probe abtastet, so dass die Abweichung, die für die (n-1)-te Linie berechnet wurde und die auch für die n-te Linie unter der Annahme von im Wesentlichen gleichen Oberflächenkrümmungen in der (n-1)-ten und n-ten Linie gilt, in der n-ten Linie Null ist.

4. Ultraschall-Bildgebungssystem nach einem der Ansprüche 1 bis 3,
wobei die Verarbeitungseinheit (35) dazu eingerichtet ist:
ein Tracking-Gate zu erzeugen, und
die Abweichung durch Vergleichen der Zeit von einem Startpunkt des Tracking-Gates, bis eine aktuell gemessene Spannungswellenform einen Spitzenwert hat, und der Zeit vom Startpunkt des Tracking-Gates, bis eine Referenzspannungswellenform einen Spitzenwert entsprechend der Referenzzeit hat, zu vergleichen.

5. Positionssteuerverfahren, umfassend:
einen Schritt zum Berechnen und Speichern einer Abweichung in einer (n-1)-ten Linie zwischen der Zeit vom Senden einer Ultraschallwelle von einer Ultraschallsondeneinheit (1) zu einer Probe (4) bis zum Empfang einer reflektierten Welle und einer Referenzzeit, wenn die Ultraschallsondeneinheit (1) die (n-1)-te Linie einer Oberfläche der Probe in einer ersten Oberflächenrichtung der Probe abtastet, wobei die Referenzzeit die Zeit vom Senden der Ultraschallwelle bis zum Empfang der reflektierten Welle ist, wenn der Abstand zwischen der Ultraschallsondeneinheit und der Oberfläche der Probe gleich der Brennweite der Ultraschallsondeneinheit ist;
einen Schritt (S505) zum Bewegen der Ultraschallsondeneinheit um einen vorgegebenen Intervallabstand in einer zweiten Oberflächenrichtung senkrecht zur ersten Oberflächenrichtung, um eine n-te Linie der Oberfläche der Probe in der Oberflächenrichtung entgegengesetzt zur ersten Oberflächenrichtung abzutasten;
einen Schritt (S502) zum Bestimmen einer Position der Ultraschallsondeneinheit in einer Richtung senkrecht zu der Oberfläche der Probe, wenn die Ultraschallsondeneinheit die n-te Linie der Oberfläche der Probe abtastet, basierend auf der gespeicherten Abweichung in der (n-1)-ten Linie;
einen Schritt zum Abtasten der n-ten Linie der Oberfläche der Probe durch die Ultraschallsondeneinheit, während die Position der Ultraschallsondeneinheit auf die bestimmte Position gesteuert wird; und
Zurückkehren (S505) zum obigen Berechnungs- und Speicherschritt, um die Abweichung in der n-ten Linie zwischen der Zeit vom Senden einer Ultraschallwelle bis zum Empfang einer reflektierten Welle und der Referenzzeit zu berechnen, wenn die Ultraschallsondeneinheit die n-te Linie der Oberfläche der Probe abtastet, und um die berechnete Abweichung zu speichern.

6. Positionssteuerverfahren nach Anspruch 5,
wobei im Schritt zum Bestimmen einer Position der Ultraschallsondeneinheit (1), wenn die Ultraschallsondeneinheit die n-te Linie der Oberfläche der Probe (4) abtastet, die Position der Ultraschallsondeneinheit in einer Richtung senkrecht zur Oberfläche der Probe (4) so bestimmt wird, dass die Abweichung, die für die (n-1)-te Zeile berechnet wurde und die unter der Annahme im Wesentlichen gleicher Oberflächenkrümmungen in der (n-1)-ten und n-ten Zeile auch für die n-te Zeile gilt, in der n-ten Zeile Null ist.

## Revendications

1. Système d'imagerie par ultrasons, comprenant :
une unité (1) de sonde ultrasonore qui est configurée pour transmettre une onde ultrasonore jusqu'à une surface d'un échantillon (4) et recevoir une onde réfléchie depuis la surface de l'échantillon ;
un dispositif (3) de commande de position ; et
un dispositif (2) de commande ;
dans lequel le dispositif de commande de position comprend :
une unité (37) de stockage configurée pour stocker un écart entre la durée depuis la transmission d'une onde ultrasonore depuis l'unité de sonde ultrasonore sur l'échantillon jusqu'à ce qu'une onde réfléchie soit reçue et une durée de référence, la durée de référence étant la durée depuis la transmission de l'onde ultrasonore jusqu'à ce que l'onde réfléchie soit reçue lorsque la distance entre l'unité de sonde ultrasonore et la surface de l'échantillon est égale à la distance focale de l'unité de sonde ultrasonore ;
une unité (35) de traitement configurée pour déterminer une position de l'unité de sonde ultrasonore dans un sens perpendiculaire à la surface de l'échantillon et pour calculer un écart entre la durée depuis la transmission d'une onde ultrasonore jusqu'à ce qu'une onde réfléchie soit reçue et la durée de référence ;
dans lequel l'unité de stockage est configurée pour stocker l'écart dans une (n-1)^{ème} ligne tel que précédemment calculé par l'unité de traitement lorsque l'unité de sonde ultrasonore balaye la (n-1)^{ème} ligne d'une surface d'un échantillon dans un premier sens de surface de l'échantillon avant que l'unité de sonde ultrasonore ne se déplace d'un pas prédéterminé dans un deuxième sens de surface perpendiculaire au premier sens de surface afin de balayer une n^{ème} ligne de la surface de l'échantillon dans le sens de surface opposé au premier sens de surface ; et
dans lequel l'unité de traitement est configurée pour déterminer la position de l'unité de sonde ultrasonore dans le sens perpendiculaire à la surface de l'échantillon lorsque l'unité de sonde ultrasonore balaye la n^{ème} ligne de la surface de l'échantillon sur la base de l'écart dans la (n-1)^{ème} ligne stocké dans l'unité de stockage,
dans lequel le dispositif de commande est configuré pour commander la position de l'unité de sonde ultrasonore dans un sens perpendiculaire à la surface de l'échantillon à la position déterminée par le dispositif de commande de position lorsque l'unité de sonde ultrasonore balaye la n^{ème} ligne de la surface de l'échantillon ; et
dans lequel l'unité de traitement est configurée pour calculer un écart dans la n^{ème} ligne entre la durée depuis la transmission d'une onde ultrasonore jusqu'à ce qu'une onde réfléchie soit reçue et la durée de référence lorsque l'unité de sonde ultrasonore balaye la n^{ème} ligne de la surface de l'échantillon, et pour stocker l'écart calculé dans l'unité de stockage.

2. Système d'imagerie par ultrasons selon la revendication 1,
dans lequel l'unité (35) de traitement est configurée pour calculer l'écart pour chaque point de mesure défini à intervalles de temps prédéterminés, et pour amener l'unité (37) de stockage à stocker l'écart.

3. Système d'imagerie par ultrasons selon la revendication 1 ou 2,
dans lequel l'unité (35) de traitement est configurée pour déterminer la position de l'unité (1) de sonde ultrasonore dans un sens perpendiculaire à la surface de l'échantillon (4) lorsque l'unité de sonde ultrasonore balaye la n^{ème} ligne de la surface de l'échantillon, de telle sorte que l'écart qui a été calculé pour la (n-1)^{ème} ligne et s'applique à la n^{ème} ligne également sous la supposition de courbures de surfaces sensiblement égales dans les (n-1)^{ème} et n^{ème} lignes, est zéro dans la n^{ème} ligne.

4. Système d'imagerie par ultrasons selon l'une quelconque des revendications 1 à 3,
dans lequel l'unité (35) de traitement est configurée pour :
générer une porte de poursuite, et
calculer l'écart en comparant une durée depuis un point de départ de la porte de poursuite jusqu'à ce qu'une forme d'onde de tension réellement mesurée ait un pic, et une durée depuis le point de départ de la porte de poursuite jusqu'à ce qu'une forme d'onde de tension de référence ait un pic correspondant à ladite durée de référence.

5. Procédé de commande de position, comprenant :
une étape de calcul et de stockage d'un écart dans une (n-1)^{ème} ligne entre une durée depuis la transmission d'une onde ultrasonore depuis une unité (1) de sonde ultrasonore sur un échantillon (4) jusqu'à ce qu'une onde réfléchie soit reçue et une durée de référence lorsque l'unité (1) de sonde ultrasonore balaye la (n-1)^{ème} ligne d'une surface de l'échantillon dans un premier sens de surface de l'échantillon, la durée de référence étant la durée depuis la transmission de l'onde ultrasonore jusqu'à ce que l'onde réfléchie soit reçue lorsque la distance entre l'unité de sonde ultrasonore et la surface de l'échantillon est égale à la distance focale de l'unité de sonde ultrasonore ;
une étape (S505) de déplacement de l'unité de sonde ultrasonore d'un pas prédéterminé dans un deuxième sens de surface perpendiculaire au premier sens de surface afin de balayer une n^{ème} ligne de la surface de l'échantillon dans le sens de surface opposé au premier sens de surface ;
une étape (S502) de détermination d'une position de l'unité de sonde ultrasonore dans un sens perpendiculaire à la surface de l'échantillon lorsque l'unité de sonde ultrasonore balaye la n^{ème} ligne de la surface de l'échantillon sur la base de l'écart stocké dans la (n-1)^{ème} ligne ;
une étape de balayage de la n^{ème} ligne de la surface de l'échantillon par l'unité de sonde ultrasonore tout en commandant la position de l'unité de sonde ultrasonore à la position déterminée ; et
le retour (S505) à l'étape de calcul et de stockage ci-dessus afin de calculer l'écart dans la n^{ème} ligne entre la durée depuis la transmission d'une onde ultrasonore jusqu'à ce qu'une onde réfléchie soit reçue et la durée de référence lorsque l'unité de sonde ultrasonore balaye la n^{ème} ligne de la surface de l'échantillon, et de stocker l'écart calculé.

6. Procédé de commande de position selon la revendication 5,
dans lequel, à l'étape de détermination d'une position de l'unité (1) de sonde ultrasonore lorsque l'unité de sonde ultrasonore balaye la n^{ème} ligne de la surface de l'échantillon (4), la position de l'unité de sonde ultrasonore dans un sens perpendiculaire à la surface de l'échantillon (4) est déterminée de telle sorte que l'écart qui a été calculé pour la (n-1)^{ème} ligne et s'applique à la n^{ème} ligne également sous la supposition de courbures de surfaces sensiblement égales dans les (n-1)^{ème} et n^{ème} lignes, est zéro dans la n^{ème} ligne.
